# EUROPÄISCHE PATENTSCHRIFT

(11) **EP 2 962 651 B1**
(45) Veröffentlichungstag und Bekanntmachung des Hinweises auf die Patenterteilung: **19.12.2018**
(21) Anmeldenummer: 15172142.0
(22) Anmeldetag: 15.06.2015
(51) Int. Cl.: A61B 17/70, A61B 17/88, A61B 17/00

(54) **MEDIZINISCHER SCHRAUBENDREHER UND SCHAFT FÜR DEN MEDIZINISCHEN SCHRAUBENDREHER**
MEDICAL SCREWDRIVER AND SHAFT FOR THE MEDICAL SCREWDRIVER
TOURNEVIS MÉDICAL ET ARBRE POUR TOURNEVIS MÉDICAL

(30) Priorität: 01.07.2014 DE 102014109200
(43) Veröffentlichungstag der Anmeldung: 06.01.2016
(73) Patentinhaber: AESCULAP AG, 78532 Tuttlingen (DE)
(72) Erfinder: Fischer, Kay, 78532 Tuttlingen (DE); Krüger, Sven, 78647 Trossingen (DE)
(74) Vertreter: Winter, Brandl, Fürniss, Hübner, Röss, Kaiser, Polte - Partnerschaft mbB

(56) Entgegenhaltungen:
- WO-A1-2014/062694
- DE-A1-102011 050 996
- US-A1- 2005 228 400
- US-A1- 2009 264 895

## Beschreibung

Die Erfindung betrifft einen medizinischen oder chirurgischen Schraubendreher gemäß dem Oberbegriff des Patentanspruchs 1, sowie einen Schaft für einen solchen Schraubendreher.

### Hintergrund der Erfindung

Pedikelschraubendreher der einschlägigen Gattung bestehen im Wesentlichen aus einer Außenhülse zum Greifen und Führen des Instruments, einer davon drehbar und verschiebbar aufgenommenen Zwischenhülse, welche mit einer Tulpe einer Pedikelschraube in Eingriff gebracht wird, sowie einem in die Zwischenhülse eingeführten, mehrteiligen Schaft, über den die Pedikelschraube schließlich verschraubt wird.

### Stand der Technik

Aus der Offenbarung der Druckschrift US 2008/0200918 A1 ist ein Schraubendreher mit einer äußeren Schutzhülse bekannt, durch deren Distalöffnung eine Zwischenhülse eingeführt ist. Durch eine Distalöffnung der Zwischenhülse wiederum ist ein Schaft eingeführt, mittels dessen eine Pedikelschraube geschraubt wird. In axialer Verlängerung der Schutzhülse an ihrem Proximalende ist eine Verriegelungseinrichtung auf den Schaft aufgeschoben, welche den Schaft mit der Zwischenhülse drehfest verbinden kann, so dass nach dem Anbringen der Pedikelschraube an den Schraubendreher ein versehentliches Lösen dieser Verbindung vermieden wird. Demnach ist das bekannte Schraubendrehersystem derart konzipiert, dass es sukzessive von außen nach innen zusammengesetzt wird, indem jedes nachfolgende zu montierende Bauteil in das bereits montierte Bauteil über dessen Distalöffnung eingeführt wird. Folglich kann einen Demontage nur in umgekehrter Reihenfolge und Richtung erfolgen, also derart, dass das Schraubendrehersystem zunächst von der Pedikelschraube zu trennen ist, damit als erstes der Schaft abgenommen werden kann, um anschließend die Verriegelungseinrichtung und schließlich die Zwischenhülse demontieren zu können.

Die Druckschrift WO 2011/043799 beschreibt einen ähnlichen Schraubendreher der oben beschriebenen Art zum Verschrauben von Pedikelschrauben. Bei diesem System wird zunächst eine Zwischenhülse dreh- und verschiebbar in eine Außenhülse über deren Proximalöffnung eingeführt. Von der Zwischenhülse ist ein zweiteiliger, zunächst demontierter Schaft aufgenommen. Ein proximaler Abschnitt des Schafts wird zunächst über eine Proximalöffnung der Zwischenhülse in diese eingeführt. Anschließend wird über eine Distalöffnung der Zwischenhülse ein zweiter Abschnitt des Schafts in diese eingeführt und mit dem ersten Abschnitt drehfest gekoppelt. Erst jetzt kann dieses System dazu verwendet werden, um eine Pedikelschraube anzubringen. Dann wird ein distales Gewinde der Zwischenhülse mit einer Tulpe einer Pedikelschraube verschraubt und dabei das Schraubeneingriffselement dazwischen eingeklemmt. Nachdem die Knochenschraube mit dem Schraubendreher in den Pedikel eingeschraubt wurde, wird die Zwischenhülse über einen proximalen Griff aus der Tulpe geschraubt und der gesamte Schraubendreher kann entfernt werden, während die Pedikelschraube im Körper verbleibt.

Die Druckschrift US 2009/264895 A1 offenbart ein medizinisches Schraubendrehersystem bei dem ein Schaft in einer Hülse geführt ist. Am distalen Endabschnitt des Schaftes ist ein Torx-Profil ausgebildet und an seinem proximalen Endabschnitt sind ein Kopplungsabschnitt für ein Drehmomentaufbringungsteil und Kopplungsabschnitt für ein Zufuhrsystem vorgesehen. Zudem weist der Schaft einen Durchgangskanal auf. Weiterhin kann die Hülse in eine Tulpe einer Pedikelschraube geschraubt werden.

Üblicherweise werden gesetzte Pedikelschrauben durch Sichtprüfung beziehungsweise Röntgenaufnahmen überprüft und bei Bedarf nachgebessert. Hierbei stellen sich einem Operateur zwei Probleme. Einerseits ist die Sicht auf die Pedikelstelle durch das vergleichsweise sperrige System bestehend aus zwei Hülsen mit jeweils einem proximal befindlichen Griff sowie dem darin aufgenommenen Schaft stark eingeschränkt. Zudem sind unter vielen anderen Ursachen eine Vielzahl von vergleichsweise sperrigen Elementen wie die des Schraubendrehers Ursache für unerwünschte Artefaktbildungen beim Röntgen, worunter die Qualität der Aufnahme leidet und die Beurteilung des Resultats erschwert wird. Da im Falle der bekannten Systeme der Schraubendreher nur als Ganzes abgekoppelt und aus dem Körper entfernt werden kann, müsste folglich ebenso die vorhandene Verbindung zwischen Pedikelschraube und Schraubendreher gelöst werden. Folglich muss bei einer erforderlichen Nachbesserung der Schraubendreher erneut mit der Pedikelschraube gekoppelt werden, was sich nachteilig auf die bereits eingeschraubte Knochenschraube und deren Verbindung mit dem Knochen auswirkt und zudem viel Zeit in Anspruch nimmt.

Häufig erfolgt nach dem Einschrauben einer Pedikelschrauben eine Zementaugmentation mittels kanülierter Schrauben zur Verbesserung ihrer Fixierung im Knochen, insbesondere zum Fixieren im osteoporotischen Wirbelkörper. Hierbei ist bei den bekannten Systemen nachteilig, dass der Schraubendreher zunächst demontiert und ein Zementapplikator an die Knochenschraube angekoppelt, eine Injektionskanüle an der Pedikelschraube befestigt oder eine weitere Hülse in die Pedikelschraube eingeführt werden muss, derart, dass diese Verbindung dem beim Zementieren entstehenden Druck Stand hält. Damit wird der Eingriff zeitlich in die Länge gezogen und es sind weitere kostenintensive Instrumente notwendig.

Nach dem Setzen von zwei oder mehr Pedikelschrauben und gegebenenfalls Zementieren ist es häufig erforderlich, dass die jeweiligen Träger der Pedikelschrauben durch Distrahieren oder Komprimieren zueinander repositioniert werden und ein Zugang zum OP-Bereich freigehalten wird, so dass der Operateur soweit wie möglich ungehindert arbeiten kann. Angriffspunkte, um Manipulationen durchzuführen sind der Hals der Knochenschraube, die Außenseite des Bodys bzw. Tulpe, die Innenseite des Bodys bzw. Tulpe oder der Torx (Inbus) in der Knochenschraube bzw. Kombinationen daraus. Manipulationen, die am Body oder Schraubenhals angreifen, sind nicht parallel. Meist ist jedoch eine parallele Distraktion / Kompression gewünscht. Eine parallele Bewegung ist nur dann gewährleistet, wenn die Knochenschraube direkt und starr mit einem Paralleldistraktor / -kompressor verbunden ist. Einige Systeme bieten die Möglichkeit, ohne den Verbindungsstab und die Klemmschraube bzw. Set-Screw einzusetzen, die Knochenschraube zum Body zu blockieren, indem sie direkt auf das Insert drücken, um dann am Body anzugreifen und eine parallele Distraktion / Kompression durchführen zu können. Diese Vorgehensweise gestaltet sich jedoch wesentlich komplexer und ist für eine schnelle Distraktion / Kompression ungeeignet.

Zudem ist bei dem zuletzt genannten System insbesondere hinsichtlich eines gefühlvollen und präzisen Einschraubens nachteilig, dass kein durchgehender Schaft verfügbar ist. Daher muss beim Einschrauben ein gewisses Maß an Spiel stets in Kauf genommen werden.

Da mit bekannten Systemen sowohl die Überprüfbarkeit der bereits angebrachten Schrauben beschränkt ist als auch anschließende und meist dringend notwendige Schritte wie Zementaugmentation und Distraktion / Kompression durch Demontieren und Montieren anderer Werkzeuge nur umständlich und zeitintensiv möglich sind, besteht Handlungsbedarf in der Weiterentwicklung der Schraubensysteme.

Vor diesem Hintergrund liegt der Erfindung die Aufgabe zu Grunde, einen spielfreien und zur Zementaugmentation geeigneten multifunktionalen Schaft, einen multifunktionale Schraubendreher sowie ein vereinfachtes und zeitsparendes Verfahren bereitzustellen, so dass Pedikelschrauben zuverlässig und präzise mit möglichst wenig Werkzeugwechsel in einen Pedikel eingedreht und dauerhaft fixiert werden können.

Diese Aufgabe wird hinsichtlich des Schraubendrehers mit den Merkmalen des Patentanspruchs 1 und hinsichtlich des Schafts mit den Merkmalen des nebengeordneten Patentanspruches gelöst.

Der medizinische bzw. chirurgische Schraubendreher gemäß Patentanspruch 1 löst die Aufgabe insbesondere dadurch, dass die Hülse mehrteilig, insbesondere zweiteilig, ausgebildet ist und einen ersten distal angeordneten Hülsenabschnitt mit einem ersten Eingriffsabschnitt und einen zweiten dazu proximal angeordneten Hülsenabschnitt mit einem komplementären zweiten Eingriffsabschnitt aufweist. Die beiden Eingriffsabschnitte sind derart konfiguriert, dass beide miteinander in Eingriff bringbar sind, so dass der zweite Hülsenabschnitt auf den ersten Hülsenabschnitt ein Drehmoment übertragen kann, um den am ersten Hülsenabschnitt vorgesehenen Kopplungsabschnitt mit der Tulpe koppeln zu können. Andererseits lassen sich die beiden Hülsenabschnitte, genauer gesagt die beiden Eingriffsabschnitte, außer Eingriff bringen, und zwar werkzeuglos, wobei lediglich der zweite Hülsenabschnitt von dem ersten Hülsenabschnitt in axialer Richtung abgezogen werden kann. Man kann also den zweiten Hülsenabschnitt von dem Schaft in proximaler Richtung abziehen und vom Schaft entfernen, ohne den ersten Hülsenabschnitt von dem Body oder der Tulpe der Pedikelschraube lösen zu müssen und ohne die Verspannung von Schraubendreher und Pedikelschraube aufgeben zu müssen.

Mit anderen Worten, der erfindungsgemäße Schraubendreher weist eine teilbare Hülse auf. Jedem Hülsenabschnitt kommt eine unterschiedliche Funktion zu. Der erste Hülsenabschnitt, der klein dimensioniert werden kann, dient lediglich zum Koppeln des Schraubendrehers mit der Tulpe der Pedikelschraube. Der zweite Hülsenabschnitt, der zum Anziehen und Verspannen des ersten Hülsenabschnitts mit der Tulpe bzw. der Pedikelschraube verwendet wird und entsprechend größer dimensioniert werden muss, in der Regel einen Greifabschnitt mit größeren radialen Abmessungen aufweisen kann, um ihn mit der Hand besser greifen und drehen zu können, kann nach dem Verspannen entfernt werden, um dem Operateur eine bessere Sicht auf die Pedikelschraube bzw. dem OP-Bereich zu ermöglichen.

Indem der vorliegende Schraubendreher eine einteilige, aus dem Stand der Technik bekannte Zwischenhülse durch eine zweiteilige, aus einer Klemmhülse (erster Hülsenabschnitt) und einer Spannhülse (zweiter Hülsenabschnitt) bestehende Zwischenhülse ersetzt, kann in vorteilhafter Weise die Spannhülse zu jedem Zeitpunkt demontiert werden. Dann verbleiben lediglich die Klemmhülse und der über die Klemmhülse mit der Pedikelschraube verspannte Schaft, welche einen deutlich geringeren Durchmesser als der gesamte Schraubendreher aufweisen und folglich weniger sperrig sind, so dass die Sicht für den Operateur auf die Eingriffsstelle verbessert wird. Ebenso wird die Artefaktbildung oder anders gesagt, werden künstlich herbeigeführte Kausalzusammenhänge auf Röntgenbildern, die durch Fehler bei der Datenerhebung entstehen, reduziert, indem weniger Bauelemente die Bildgebung beeinflussen können. Auf diese Weise kann schneller eine Diagnose erstellt werden und die Qualität wird ebenso verbessert.

Weiterhin geht mit der zweiteiligen Zwischenhülse der besondere Vorteil einher, dass selbst bei Demontage der Spannhülse die Klemmhülse montiert bleiben kann und folglich die steife, verspannte Verbindung zwischen Klemmhülse und Tulpe einerseits und zwischen Schaft und Pedikelschraubenkopf andererseits aufrechterhalten bleibt. Dies spart jedenfalls dann Zeit, wenn die Knochenschraube nachgebessert werden müsste, denn dann muss lediglich der Schaft rotiert werden, ohne dass eine erneute Koppelung zwischen Pedikelschraube und Schaft / Schraubendrehersystem erforderlich ist. Ebenso ist daran vorteilhaft, dass, wenn die Knochenschraube endgültig eingesetzt ist, die Spannhülse einfach auf den Schaft aufgeschoben und der zweite Eingriffsabschnitt der Spannhülse mit dem ersten Eingriffsabschnitt der Klemmhülse gekoppelt werden kann, um den Schaft von der Tulpe zu entkoppeln.

Ganz besonders vorteilhaft daran, dass nach Demontage der Spannhülse der Schaft mit der Klemmhülse im verspannten Zustand mit der Pedikelschraube stehen bleibt, ist, dass daran nahezu jede Art von Distraktoren oder Kompressoren und insbesondere Paralleldistraktoren und -kompressoren angeschlossen und erforderliche Manipulationen durchführt werden können. Damit kann nach dem Einschrauben der Pedikelschraube der Operateur bei nahezu freier Sicht auf den OP-Bereich mittels Distraktor oder Kompressor Kräfte und / oder Drehmomente in jede beliebige Richtung auf die Knochenschraube und damit auf den Knochen übertragen. Somit kann nach dem Entfernen der Spannhülse der Distraktor oder Kompressor unmittelbar an dem einteiligen Schraubendreherschaft angreifen und nicht an einer darüber angeordneten ggf. mit erheblichen radialen und axialen Spiel versehenen Hülse wie beim Stand der Technik. Somit kann eine wesentliche feinfühligere Manipulation der Pedikelschraube bzw. des Wirbelkörpers erfolgen.

Damit erfüllt der erfindungsgemäße Schraubendreher gleichzeitig die Funktion eines Schraubendrehers wie auch die eines Kraftübertragungsglieds, an das nahezu jedes beliebige zur Manipulation geeignete Gerät angeschlossen werden kann. Zudem wurde ein einfaches, günstiges und ebenso leicht zu montierendes Instrument geschaffen, das durch seine Multifunktionalität Zeit und Kosten spart. Außerdem wird durch die dauerhafte Koppelung zwischen Schaft und Pedikelschraube beziehungsweise Knochenschraube verhindert, dass diese verschmutzt.

Des Weiteren ist das chirurgische Instrument trotz seiner multifunktionalen und teilweise komplexen Gestaltung nahezu vollständig zerlegbar und demnach gut zu reinigen.

Vorteilhafte Weiterbildungen des Schraubendrehers sind Gegenstand der Unteransprüche.

Gemäß einem Ausführungsbeispiel können der erste und zweite Eingriffsabschnitt als korrespondierende Kronen ausgebildet sein, die in axialer Richtung in einander greifen und lediglich einen Formschluss in Umfangsrichtung zur Drehmomentübertragung bilden. Die Herstellung einer solchen Krone gestaltet sich als sehr einfach und damit kostengünstig. Ferner sind keine besonderen Formtoleranzen einzuhalten, so dass die Spannhülse jederzeit und beliebig oft mit der Klemmhülse in Eingriff bringbar ist, indem sie einfach auf den Schaft aufgeschoben wird bis die am Distalende und stirnseitig angeordnete Kronen beziehungsweise deren Zacken in Zwischenräume der Krone der Klemmhülse greifen. Die Kronen vermeiden im Übrigen eine Zunahme der Bauteilgröße in Radialrichtung, so dass zusätzlich kein negativer Einfluss auf die Sicht des OP-Feldes hinzunehmen ist. Mit anderen Worten dienen die beiden Eingriffsabschnitte der beiden Hülsenabschnitte lediglich zur Drehmomentübertragung, um eine manuell auf den zweiten Hülsenabschnitt, insbesondere an einem speziell dafür vorgesehenen Greifabschnitt, aufgebrachte Drehbewegung an den ersten Hülsenabschnitt zu übertragen und so beispielsweise einen an dem ersten Hülsenabschnitt vorgesehenen Außengewindeabschnitt in ein in der Tulpe der Pedikelschraube vorgesehenes Innengewinde zu schrauben und so eine axiale Kopplung von dem ersten Hülsenabschnitt bzw. dem Schraubendreher mit der Tulpe bzw. der Pedikelschraube zu realisieren. Das Ineingriffbringen bzw. Außereingriffbringen beider Eingriffsabschnitte erfolgt durch axiale Verschiebung der beiden Hülsenabschnitte zu- bzw. voneinander. Anstelle von Kronen ist jede andere Geometrie denkbar, die einen Formschluss in Umfangsrichtung zwischen beiden Hülsenabschnitten herstellen. Die beiden Eingriffsabschnitte können ferner so ausgestaltet sein, dass sie in axialer Richtung eine Überdeckung über eine bestimmte axiale Länge haben, um auch dann noch eine Drehmomentübertragung zu ermöglichen, wenn die beiden Hülsenabschnitte etwas näher oder etwas weiter zu einander beabstandet sind.

Der Schaft kann zwei miteinander montierbare Schaftabschnitte aufweisen und der erste Hülsenabschnitt im montierten Zustand zwischen den zwei montierten Schaftabschnitten, insbesondere axial nicht verschiebbar, gefangen sein. Dadurch wird erreicht, dass der Schaft und der ersten Hülsenabschnitt nicht nur eine funktionale Einheit zum Aufrechterhalten der Kopplung bzw. Verspannung von Schraubendreher und Pedikelschraube bilden, sondern auch eine bauliche Einheit. Dadurch wird verhindert, dass der ersten Hülsenabschnitt bzw. die Klemmhülse nicht versehentlich vom Schaft rutscht. Ferner spart sich der Operateur den Montageschritt der Klemmhülse auf den Schaft.

Gemäß einem alternativen oder zusätzlichen Ausführungsbeispiel kann der erste Hülsenabschnitt am Schaft zumindest über eine vorbestimmte Länge axial verschiebbar gelagert sein, wobei ein in distaler Richtung vor dem Kopplungsabschnitt am Schaft vorgesehener Abschlag den axialen Freiheitsgrad des ersten Hülsenabschnitts begrenzt, so dass der erste Hülsenabschnitt in distaler Richtung eine axiale Kraft auf den Schaft übertragen kann, wenn dieser mit der Tulpe der Pedikelschraube gekoppelt wird. Ein gewisses axiales Spiel der Klemmhülse erlaubt, dass der Schraubendreherabschnitt zuerst sauber und vollständig in den Schraubenkopf axial eingeführt werden kann und die Klemmhülse axial ausweichen kann, wenn das Außengewinde der Klemmhülse das Innengewinde der Tulpe kontaktiert. Dadurch erspart sich der Operateur das gleichzeitige Drehen der Spannhülse beim Einführen des Schraubendreherabschnitts in den Schraubenkopf.

Um die Handhabung des Schraubendrehers zu erleichtern, kann eine relativ zur Hülse drehbare Außenhülse zum Greifen des Schraubendrehers auf den ersten und/oder zweiten Hülsenabschnitt lösbar aufgebracht werden, insbesondere aufgeschoben werden. Über diese sich beim Drehen des Schafts oder der Spannhülse nicht mitdrehende Außen- oder Rotationshülse kann der Operateur den Schraubendreher besser führen und ausrichten.

Die Außenkonturen des ersten Hülsenabschnitts und des zweiten Hülsenabschnitts können derart ausgestaltet sein, dass die relativ dazu drehbare Außenhülse zwischen beiden Hülsenabschnitten axial gefangen bzw. axial festgelegt ist. Wenn die Außenhülse in axialer Richtung ortsfest ist, erleichtert dies die Feinjustierung des Schraubendrehers. Ferner kann der Operateur über die Außenhülse eine Axialkraft auf den Schraubendreher aufbringen.

Der Schaft kann in Axialrichtung zumindest einen durchgehenden, innen liegenden Kanal aufweisen. In diesem Fall kann der Schaft nicht nur zum Eindrehen der Pedikelschraube in den Wirbel, sondern auch noch zur Zementaugmentation verwendet werden, ohne zunächst den Schraubendreher entfernen und ein anderes Instrument mit der Pedikelschraube koppeln zu müssen. Die oben beschriebene Konstruktion der Klemmhülse, die beim Einschrauben in die Tulpe gleichzeitig den Schaft über seinen Anschlag fest verspannt, ist ferner besonders vorteilhaft, da somit eine sichere Kopplung zwischen Pedikelschraube und Kanalaustrittsöffnung am distalen Ende des Schafts geschaffen wird, die einem Druck des Zements beim Einspritzen Stand hält. Der Schaft kann somit als Injektionskanüle dienen, falls eine Zementaugmentation erforderlich ist bzw. gewollt ist.

Ein derart ausgebildeter Schraubendreher ermöglicht eine große Zeitersparnis, insbesondere wenn eine Vielzahl von Pedikelschrauben einzuschrauben und zu zementieren ist. Das liegt sowohl im Sinne des Patienten hinsichtlich seiner Gesundheit als auch im Sinne des Krankenhauses hinsichtlich OP-Zeiten.

Da der erfindungsgemäße Schraubendreher komplett demontierbar ist, insbesondere die aus Spann- und Außenhülse bestehende Drehmechanik vom Schaft entfernt werden kann, kann der Schaft, wenn er zur Zementinjektion verwendet wird, als ein Einwegelement (Single-Use-Element) ausgeführt sein. Da beim Zementieren immer Zement im Schaft verbleibt, kann der vergleichsweise einfach aufgebaute und damit kostengünstigere Schaft aufgrund von nicht vermeidbaren und nur äußerst aufwendig zu entfernenden Zementrückständen entsorgt werden, während die anderen, komplexeren und teureren Elemente wiederverwendet werden können (Multi-Use-Element).

Um eine sichere und saubere Verbindung zwischen dem als Injektionskanüle fungierenden Schaft bzw. dem darin vorgesehenen Kanal und einem Zementapplikator herzustellen, kann der Schaft ferner an seinem proximalen Endabschnitt ein Verbindungssystem, vorzugsweise eine Luer-System aufweisen. Die Verwendung dieser genormten Schnittstelle ermöglicht eine noch einfachere und schnellere Verbindung der Injektionskanüle mit dem Zementapplikator, so dass die bereits eingeschraubte, kanülierte Knochenschraube im Knochen zementiert werden kann. Dabei spielt es keine besondere Rolle, ob ein Luer-Lock, -Ansatz, -Steck oder -Slip verwendet wird. Selbstverständlich kann der Schaft andere gängige Kupplungsabschnitte aufweisen, solange diese nicht ein proximales Entfernen der Spannhülse behindern.

Der zweite Hülsenabschnitt am Schaft kann zur axialen Sicherung lösbar arretierbar sein. Dadurch wird sichergestellt, dass der zweiten Hülsenabschnitt bzw. die Spannhülse beim Drehen nicht versehentlich axial verschoben wird und die beiden Eingriffsabschnitte außer Eingriff kommen. Dabei kann insbesondere ein am zweiten Hülsenabschnitt vorgesehenes in radialer Richtung vorgespanntes Riegelelement vorgesehen sein, dass mit einer am Schaft ausgebildeten Umfangsnut selbsttätig in Eingriff kommt und einen axialen Formschluss bildet. Diese Verriegelung kann manuell, z. B. mittels eines Druckknopfs, lösbar sein.

Um ein versehentliches Lösen der Kopplung zwischen Schraubendreher und Pedikelschraube durch Drehen der Spannhülse zu verhindern, kann eine Drehsperreinrichtung, insbesondere ein Sperrschieber, vorgesehen sein, die zwischen einer Drehposition, in der der zweite Hülsenabschnitt relativ zum Schaft drehbar ist, und einer Sperrposition, in der der zweite Hülsenabschnitt nicht relativ zum Schaft drehbar ist, bringbar ist. Diese kann Drehsperreinrichtung kann also nach erfolgter Kopplung und Verspannung zwischen Schraubendreher und Pedikelschraube entsprechend in die Sperrposition gebracht werden.

Die der Erfindung zugrunde liegenden Aufgabe wird auch durch einen erfindungsgemäßen Schaft gelöst, der sich für einen medizinischen Schraubendreher nach einem der zuvor genannten Ausführungsbeispiele eignet. Ein solcher Schaft weist im Bereich seines proximalen Endabschnitts einen Kopplungsabschnitt für ein abnehmbares Handhabungsteil zur Drehmomentaufbringung auf den Schaft auf und an dessen distalen Endabschnitt ist ein Schraubendreherabschnitt, insbesondere mit einem Torx- oder Inbus-Profil, vorgesehen, der in eine entsprechende Aufnahme am Schraubenkopf der Pedikelschraube einführbar ist und ein auf den Schaft aufgebrachtes Drehmoment auf den Schraubenkopf übertragen kann. Dabei weist der Schaft ferner in Axialrichtung einen durchgehenden, innen liegenden Kanal auf, wobei am proximalen Endabschnitt des Schafts ein Verbindungssystem, vorzugsweise ein Luer-System, zum Anschließen eines Zementapplikators an dem Kanal vorgesehen ist. Zudem ist eine mit einer Tulpe der Pedikelschraube koppelbaren Klemmhülse an dem Schaft derart angeordnet, dass die Klemmhülse und der Schaft eine bauliche Einheit bilden.

Ein solcher Schaft ist insbesondere multifunktional einsetzbar und eignet sich nicht nur zum Eindrehen einer Knochen- oder Pedikelschraube, sondern auch zum Injizieren von Zement über den innenliegenden Kanal in die Knochen bzw. Pedikelschraube. Somit ist kein Instrumentenwechsel notwendig, was dem Operateur nicht nur Zeit spart, sondern auch den chirurgischen Eingriff vereinfacht, da kein zweites Instrument mit der Pedikelschraube gekoppelt werden muss.

### Kurze Beschreibung der Zeichnungen

Es zeigen:
Figur 1 eine Querschnittsansicht eines Schraubendrehersystems gemäß einer bevorzugten Ausführungsform der Erfindung;
Figur 2A eine perspektivische Ansicht des Schraubendrehersystems gemäß der bevorzugten Ausführungsform der Erfindung;
Figur 2B eine perspektivische Ansicht des Schraubendrehersystems mit demontierter Drehmechanik gemäß der bevorzugten Ausführungsform der Erfindung;
Figur 3 eine teilweise geschnittene Teilseitenansicht des Schraubendrehersystems mit einer polyaxialen Pedikelschraube gemäß der bevorzugten Ausführungsform der Erfindung;
Figuren 4A und 4B jeweils in einer Draufsicht das in einen Wirbel eingeschraubte Schraubendrehersystem ohne und mit Drehmechanik; und
Figuren 5A und 5B jeweils in einer perspektivischen Ansicht zwei Schraubendrehersysteme mit demontierter Drehmechanik, die jeweils in einen Wirbel eingeschraubt sind, ohne und mit Koppelung eines beispielhaften Paralleldistraktors / Parallelkompressors daran.

### Detaillierte Beschreibung einer bevorzugten Ausführungsform

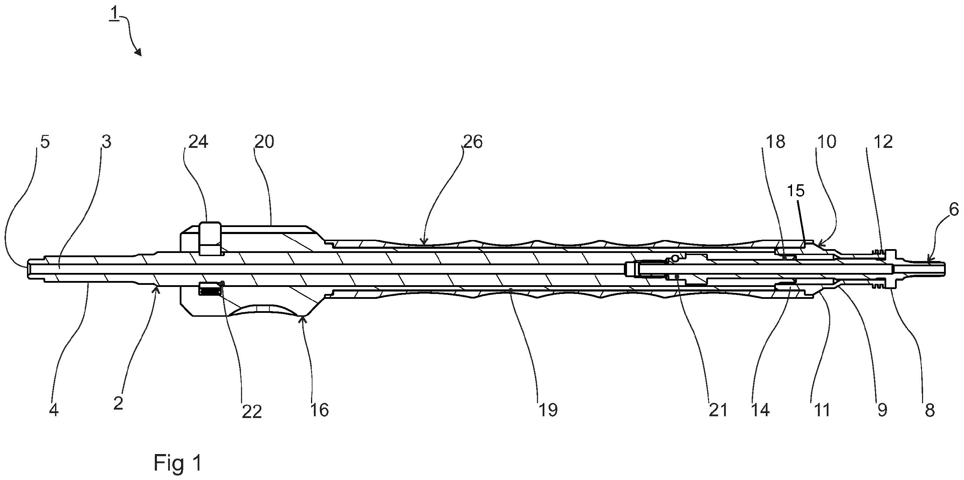

In Figur 1 ist ein Schraubendrehersystem 1 mit einem Schaft 2 dargestellt, der an seinem einen axialen bzw. proximalen Ende (linke Seite in Figur 1) einen mit einem Handgriff (nicht dargestellt) koppelbaren, als Mehrkantprofil ausgeführten Kopplungsabschnitt 4 und an seinem anderen axialen bzw. distalen Ende (rechte Seite in Figur 1) einen zur Drehmomentübertragung geeigneten, vorzugsweise als Torx-Profil oder Inbusprofil ausgeführten, Schraubendreherabschnitt 6 aufweist. Diesem schließt sich proximal ein gegenüber einer Außenmantelfläche des Schafts 2 radial erweiterter Anschlag 8 an. Dem Mehrkantprofil 4 schließt sich proximal ein genormtes Verbindungssystem, vorzugsweise ein Luer-System 5 an. Ferner ist der Schaft 2 entlang seiner Längsachse von einem Durchgangskanal oder Axialbohrung 3 durchsetzt.

Wie ferner Figur 1 zeigt, sind zwei Bauteile an einer Fügestelle 21 zum Schaft 2 integriert. Hierbei ist wesentlich, dass der Schaft 2 als Ganzes als ein integrales und spielfreies Bauteil vorliegt. Dies kann durch Werkstoffeinstückigkeit, wobei der Schaft als ein einstückiges Teil hergestellt wird, oder durch Verbinden mindestens zweier Teile mit üblichen Verbindungsmitteln erreicht werden.

Ein erster Hülsenabschnitt bzw. eine Klemmhülse 10 ist auf dem Schaft 2 koaxial angeordnet und sowohl axial verschiebbar als auch drehbar. Der axiale Freiheitsgrad der Klemmhülse 10 ist zum distalen Ende hin durch den Anschlag 8 begrenzt. Bei einem aus mehreren Teilen gefügtem Schaft erfolgt ein Anbringen der Klemmhülse 10 vorzugsweise vor der Endmontage des Schafts 2. Beim werkstoffeinstückig ausgebildeten Schaft wird die Klemmhülse über Aufschieben auf das Mehrkantprofil 4 bis hin zum Anschlag 8 montiert oder aufgeschrumpft. Am distalen Ende der Klemmhülse 10 ist ein Außengewinde 12 ausgebildet. In Proximalrichtung ist die Klemmhülse 10 über zwei kegelstumpfförmige Abschnitte 9 und 11 stufig radial erweitert. An den proximalen Abschnitt 11 dieser beiden Abschnitte schließt sich ein zur Drehmomentübertragung geeigneter, als Krone ausgebildeter erster Eingriffsabschnitt 14 an. Dieser weist einen kleineren Außendurchmesser als der proximale Abschnitt 11 auf, so dass an diesem eine als Wellenschulter ausgebildete proximale Ringstirnfläche 15 stehen bleibt. Mit anderen Worten erstrecken sich ausgehend von der proximalen Ringstirnfläche 15 in axialer Richtung mindestens drei, vorzugsweise vier gleichmäßig am Umfang verteilte Vorsprünge der Krone, die den ersten Eingriffsabschnitt 14 der Klemmhülse 10 ausbilden.

Der Schaft 2 ist koaxial von einer abnehmbaren Spannhülse 16 umgriffen. Diese ist wie die Klemmhülse 10 auf dem Schaft 2 drehbar angeordnet. Die Spannhülse 16 ist vom proximalen Ende des Schafts 2 bis zur Klemmhülse 10 aufschiebbar, wobei an ihrem distalen Ende ein zweiter Eingriffsabschnitt 18 komplementär zum ersten Eingriffsabschnitt 14 ausgebildet ist. Auch der zweite Eingriffsabschnitt 18 ist als Krone zur Drehmomentübertragung geeignet ausgebildet und als Koppelabschnitt ausgestaltet. Dabei stehen die Eingriffsabschnitte 14, 18 stirnseitig miteinander in Eingriff.

Vom zweiten Eingriffsabschnitt 18 ausgehend erstreckt sich proximal ein im Wesentlichen zylindrischer Hülsenabschnitt 19 der Spannhülse 16 mit kreisförmigem Querschnitt, der sich über einen vergleichsweise kurzen, konischen Abschnitt zu einem Griffabschnitt 20 zum Greifen und Drehen der Spannhülse 16 fortsetzt, der deutlich größere radiale Abmessungen als der Hülsenabschnitt 19 und mehrere in Umfangsrichtung vorgesehene abgeflachte Greifabschnitte oder Griffmulden aufweist.

Der Schaft 2 durchdringt den Griffabschnitt 20 so weit, dass sein Mehrkantprofil 4 aus der Spannhülse 16 hervorragt und eine Handhabe mit dem Mehrkantprofil 4 gekoppelt werden kann, um auf den Schaft 2 und folglich auf eine mit dem Torx-Profil des Eingriffsabschnitts 6 gekoppelte Schraube ein Drehmoment zu übertragen.

Wie ferner in Figur 1 gezeigt, weist der Schaft 2 in distaler Richtung beabstandet zum Mehrkantprofil 4 eine Umfangsnut 22 auf, welche sich im montieren Zustand der Spannhülse 16 im Bereich des Griffabschnitts 20 befindet. Der Griffabschnitt 20 ist an einem in Radialrichtung innen liegenden Abschnitt mit einer allgemein bekannten Arretiervorrichtung versehen, welche in die Umfangsnut 22 eingreift und somit eine formschlüssige Axialsicherung für die Spannhülse 16 auf dem Schaft 2 darstellt. Genauer gesagt handelt sich dabei um eine im Griffabschnitt 20 in Querrichtung verschieblich gelagerte und über eine Feder oder ähnliches in Querrichtung vorgespannte Verriegelung, wie z.B. eine Lochscheibe, die gelöst werden kann, indem entgegen der Federvorspannung die Verriegelung oder ein Druckknopf 24 gedrückt wird, wodurch die Arretiervorrichtung und die Umfangsnut 22 außer Eingriff gebracht werden und die Spannhülse 16 proximal von dem Schaft 2 abgezogen und entfernt werden kann.

Um das Schraubendrehersystem 1 greifen und führen zu können, ist auf die Spannhülse 16 eine rotationssymmetrische, ergonomisch geformte Rotationshülse beziehungsweise Außenhülse 26 koaxial aufgeschoben. Die Außenhülse 26 ist dabei mit jeweils einer Stirnseite distal in Anschlag mit der Klemmhülse 10 und proximal in Anschlag mit der Spannhülse 16. Genauer gesagt umschließt sie den ersten Eingriffsabschnitt 14 und steht dort mit ihrer distalen Stirnfläche an der Ringstirnfläche 15 der Klemmhülse 10 an. Die entgegengerichtete Stirnseite der Außenhülse 26 stellt einen axialen / proximalen Anschlag dar, an dem die Spannhülse 16 mit ihrem Griffabschnitt 20 anliegt. Die Rotationshülse 26 ist relativ zur Spannhülse drehbar, so dass das Schraubendrehersystem 1 gehalten werden kann, während die Spannhülse 16 oder der Schaft 2 gedreht wird. Die Rotationshülse 26 kann nach dem Entfernen der Spannhülse 16 ebenfalls in proximaler Richtung von dem Schaft 2 abgezogen und entfernt werden bzw. vor dem Aufschieben der Rotationshülse auf den Schaft 2 aufgeschoben werden.

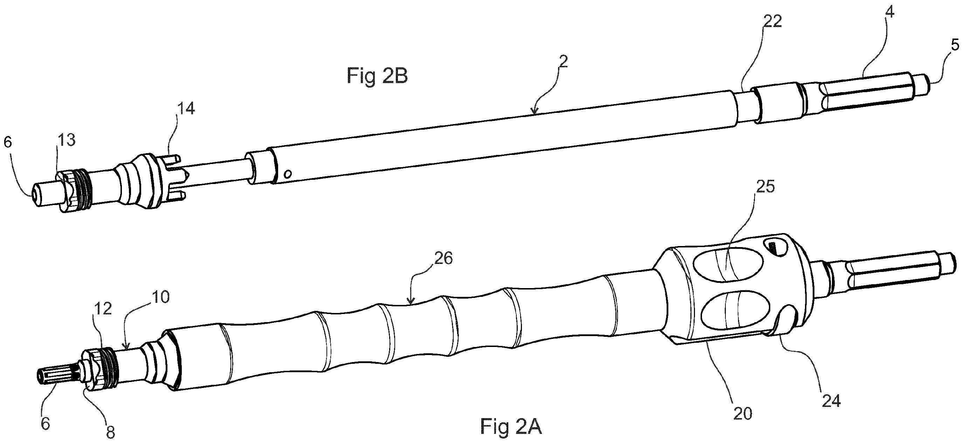

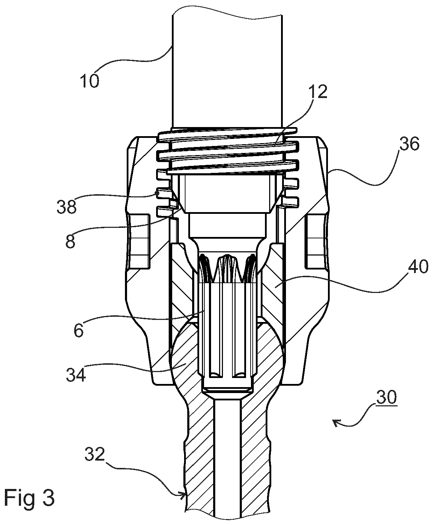

Figur 2A zeigt eine isometrische Ansicht des Schraubendrehersystems 1, in der das Mehrkantprofil 4 sowie der im Griffabschnitt 20 integrierte Druckknopf 24 verdeutlicht sind. Des Weiteren sind die zuvor bereits angesprochenen Vertiefungen oder Griffmulden 25 in den Griffabschnitt 20 vorgesehen, um eine bessere Handhabung zu erreichen. Am dazu entgegen gesetzten axialen Ende des Schafts 2 befindet sich das Torx-Profil 6, dem sich in Proximalrichtung der Anschlag 8 anschließt. Der Anschlag 8 weist an zwei in Radialrichtung einander gegenüberliegenden Seiten Abschnitte 13 mit Abflachungen bis auf die Mantelumfangsfläche des Schafts 2 auf, an denen eine bogenförmige Außenkontur ausgebildet ist, deren Außendurchmesser geringer als der des Außengewindes 12 ist. Hierbei ist im Idealfall der Außendurchmesser der Abflachung 13 höchstens so groß wie der Kerndurchmesser des Gewindeabschnitts 12. Dies hat den einen Effekt, dass das Außengewinde 12 der Klemmhülse 10 zumindest in den Bereichen der Abflachungen 13 des Anschlags 8 radial über die Klemmhülse 10 hervorragt, so dass es in ein Innengewinde 38 (siehe Figur 3) einer Tulpe 36 eingeschraubt werden kann. Außerdem greift die bogenförmige Außenkontur der Abflachungen 13 jeweils in komplementäre Konturen an Innenwänden der Tulpe 36, um so eine weitgehend spielfreie Verbindung zwischen Schaft 2 und Pedikelschraube 30 herzustellen. Dadurch wird zudem eine Radialsicherung zwischen dem Schaft 2 und der Tulpe 36 realisiert.

Weiterhin ist zu erkennen, dass die Außenhülse 26 vom Griffabschnitt 20 bis zur Wellenschulter 15 der Klemmhülse 10, die distal zum ersten Eingriffsabschnitt 14 angeordnet ist, den Schaft 2 sowie die Klemmhülse 10 und die Spannhülse 16 umgibt.

Figur 2B zeigt eine isometrische Ansicht des Schraubendrehersystems 1, bei der die Drehmechanik, also die Spannhülse 16 und die Außenhülse 26, abgekoppelt sind. Hierbei ist zudem die Umfangsnut 22 des Schafts 2 erkennbar, in die die Arretiervorrichtung (nicht dargestellt) greift und somit die Axialsicherung zwischen Schaft 2 und Spannhülse 16 realisiert.

Figur 3 zeigt eine teilweise geschnittene Teilseitenansicht eines mit einer polyaxialen Pedikelschraube 30 montierten Schraubendrehersystems 1. Die Pedikelschraube 30 setzt sich aus einer Knochenschraube 32, die vorzugsweise mit ihrem kugelförmigen Schraubenkopf 34 von einem axialen / distalen Endabschnitt der Tulpe 36 schwenkbar aufgenommen ist, und einem auf den Schraubenkopf 34 aufgesetzten Insert 40 zusammen. Indem das Außengewinde 12 der Klemmhülse 10 in das am proximalen Ende der Tulpe 36 befindliches Innengewinde 38 eingeschraubt ist, ist das Torx-Profil 6 in einen dafür vorgesehenen und korrespondierenden als Torx-Profil ausgebildeten Eingriffsabschnitt im Schraubenkopf 34 eingesteckt. Dabei wird das Torx-Profil 6 von einer axialen Durchgangsbohrung im Insert 40 geführt.

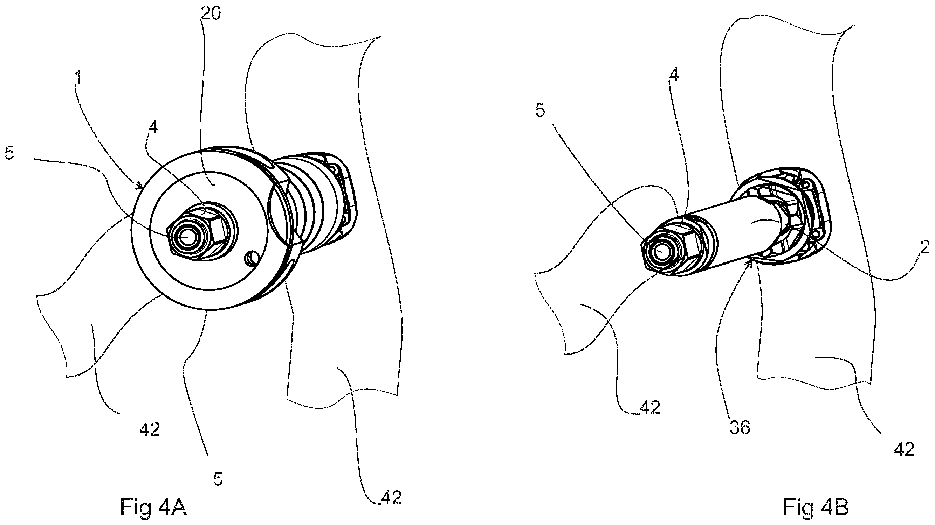

Figur 4A zeigt eine Draufsicht eines Schraubendrehersystems 1, das mit der in einen Wirbel 42 eingeschraubten Pedikelschraube 30 (nicht dargestellt) verspannt ist. Zu erkennen ist hier lediglich das Luer-System 5, das Mehrkantprofil 4 sowie eine proximale Stirnfläche des Griffabschnitts 20. Jedoch ist die Sicht im Übrigen durch den vergleichsweise sperrigen Griffabschnitt 20 der Spannhülse 16 verdeckt, wodurch es dem Operateur erschwert ist, einen Einschraubzustand der Pedikelschraube 30 in den Wirbel 42 zu kontrollieren.

Dagegen ist in Figur 4B nach dem Einschrauben der Pedikelschraube 30 in den Wirbel 42 die Drehmechanik nach proximal demontiert. Daher ist die Sicht in der Draufsicht entlang des Schafts 2 bis zur Pedikelschraube 30 frei, so dass der Operateur nahezu frei Sicht bis auf die Tulpe 36 hat.

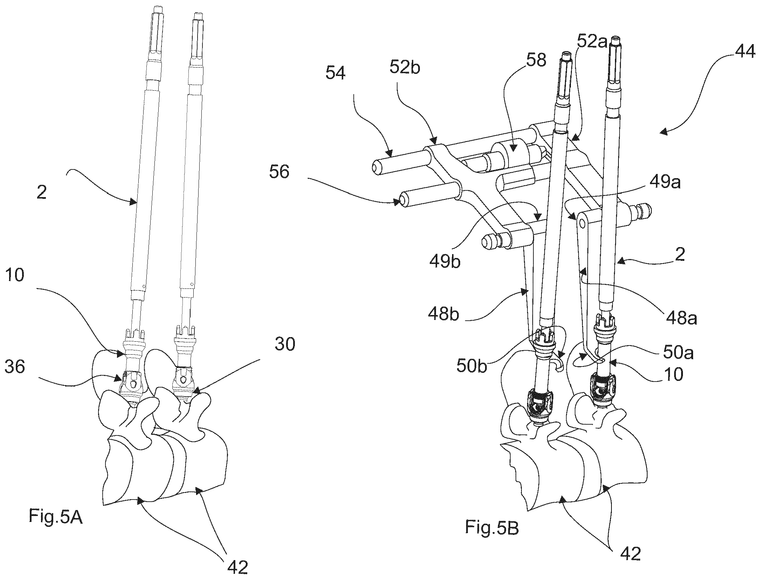

Figur 5A zeigt beispielhaft eine perspektivische Ansicht von zwei erfindungsgemäßen Schraubendrehersystemen 1 mit demontierter Drehmechanik, welche jeweils mit der in den Wirbel 42 eingeschraubten Pedikelschraube 30 verspannt sind. Diese Ansicht zeigt nochmals deutlich, wie die Klemmhülse 10 in die Tulpe 36 eingeschraubt ist und durch diese Verbindung den Schaft 2 mit der Pedikelschraube 30 verspannt.

In Figur 5B ist ein mit den beiden Schraubendrehersystemen aus Figur 5A gekoppelter Paralleldistraktor / -kompressor 44 (nachfolgend "Distraktor" genannt), welcher sowohl zur parallelen Distraktion wie auch Kompression geeignet ist, dargestellt. Der Distraktor 44 hat zwei Armabschnitte 48a und 48b, die an ihrem freien Ende jeweils über einen Koppelabschnitt bzw. Greifer 50a, 50b die Klemmhülse 10 in einem Bereich zwischen dem kegelstupfförmigen Abschnitt 9 sowie dem Außengewinde 12 umgreifen. Das andere Ende des einen Armabschnitts 48a ist über ein Gelenk 49a mit einem ersten Kraftübertragungselement 52a des Distraktors 44 verbunden und das andere Ende des anderen Armabschnitts 48b ist über ein weiteres Gelenk 49b mit einem zweiten Kraftübertragungselement 52b verbunden. Mit dem ersten Kraftübertragungselement 52a sind zwei im Wesentlichen parallel zueinander verlaufende Führungsschienen 54 und 56 verbunden, die mit ihrem einen axialen Ende jeweils mit dem im Wesentlichen senkrecht dazu verlaufenden Kraftübertragungselement 52a verbunden sind. Über das freie axiale Ende der Führungsschienen 54, 56 wird das zweite Kraftübertragungselement 52b auf die beiden Schienen 54, 56 aufgeschoben. Auf diese Weise sind die Kraftübertragungselemente 52a, 52b relativ zu einander translatorisch beweglich. Entlang der Translationsrichtung ist zwischen beiden Kraftübertragungselementen 52a, 52b ein Kraftaufbringungselement 58 zwischengeschaltet. Indem der Operateur eine Wirklänge das Kraftübertragungselement 58 mechanisch oder elektrisch (nicht dargestellt) verkürzt oder verlängert, wird eine Kraft auf die beiden Kraftübertragungselement 52a, 52b ausgeübt und deren Abstand zueinander verändert. Diese Kraft wird über die Gelenke 49a, 49b auf die Armabschnitte 48a, 48b übertragen wird. Da diese mit den Klemmhülsen 10 in Eingriff stehen, wird diese Kraft an den Angriffspunkten der Greifer 50 an die Klemmhülsen 10 auf die Klemmhülsen 10 und folglich auf die Knochen übertragen.

Es sei an dieser Stelle darauf hingewiesen, dass die Darstellungen in den Figuren 5A und 5B nur zu Darstellungszwecken auf zwei Schraubendrehersysteme beschränkt wurden. Es ist daher ebenso möglich, dass mehr als jeweils zwei mit einem Schaft 2 verspannte Pedikelschrauben 30 mit verschiedenen Knochen, vorzugsweise Wirbeln 42 verschraubt sind. Ebenso kann der Distraktor 44 mehr als zwei Armabschnitte 48a und 48b, die jeweils mit einem Schaft 2 bzw. einer Klemmhülse 10 gekoppelt sind, aufweisen.

Im Folgenden wird beschrieben, wie der erfindungsgemäße Schraubendreher 1 verwendet wird. Insbesondere wird dargestellt, dass der Schraubendreher 1 neben der Funktion als Schraubendreher gleichzeitig die Funktion einer Zementinjektionskanüle und als Kraftübertragungsglied für Distraktoren bzw. Kompressoren dient.

Um eine Pedikelschraube 30 mit dem erfindungsgemäßen Schraubendreher 1 zu koppeln, kann wie folgt vorgegangen werden:
Zunächst wird der Schaft 2 derart vorbereitet, dass die Klemmhülse 10 koaxial auf den Schaft 2 aufgeschoben und proximal vom Anschlag 8 angeordnet ist. Entweder wird dazu die Klemmhülse 10 entweder auf den Schaft 2 aufgeschoben oder ist bereits auf dem Schaft 2 vormontiert.

Anschließend wird die Außenhülse 26 auf die Spannhülse 16 gesteckt, genauer gesagt über die Krone 18 der Spannhülse 16 auf diese aufgeschoben, und beide zusammen von der proximale Seite bzw. von hinten über das Mehrkantprofil 4 soweit auf den Schaft 2 aufgeschoben, bis die Krone 18 der Spannhülse 16 und die Krone 14 der Klemmhülse 9 axial ineinander greifen und die radial innen in der Spannhülse vorgesehene Arretiervorrichtung in die Umfangsnut 22 des Schafts 2 eingreift und somit die Spannhülse 16 gegenüber dem Schaft 2 axial festlegt bzw. sichert. Die Außenhülse 26 ist dann zwischen der Spannhülse 16 und der Klemmhülse 10 axial gefangen, aber zu diesen relativ drehbar.

Alternativ kann auch zuerst die Außenhülse 26 auf den Schaft 2 aufgeschoben werden und anschließend die Spannhülse 16 auf den Schaft 2 und in die Außenhülse 26 geschoben werden.

Wird nun der Schraubendrehabschnitt 6 in eine entsprechende Schraubendreheraufnahme (z.B. Torx oder Inbus) im Schraubenkopf 34 der Pedikelschraube 30 derart eingesetzt, dass der Anschlag 8 zwischen den beiden Flanken der Tulpe 36 eintaucht, und dabei die Spannhülse 16 und über diese die Klemmhülse 10 gedreht, schraubt sich der am distalen Ende der Klemmhülse 10 vorgesehene Außengewindeabschnitt 12 in das entsprechende Innengewinde 38 der Tulpe 36 der Pedikelschraube 30. Da der axiale Freiheitsgrad der Klemmhülse 10 beim Einschrauben in die Tulpe 36 durch den am distalen Ende des Schafts vorgesehenen Anschlag 8 begrenzt ist und das distale Ende des Schraubendrehabschnitts 6 gegen den Boden der im Schraubenkopf 34 ausgebildeten Schraubendreheraufnahme drückt, bewirkt das weitere Eindrehen der Klemmhülse 10 in die Tulpe 36, dass sich die Tulpe 36 vom Schraubenkopf 34 zunehmend entfernt und schließlich Tulpe 36 und Schraubenkopf 34, Klemmhülse 10 und Tulpe 36 und Schraubendrehabschnitt 6 und Schraubenkopf 6 spielfrei miteinander verspannt werden.

Vorzugsweise ist die Klemmhülse 10 auf dem Schaft 2 nicht nur drehbar, sondern auch derart axial beweglich gelagert, dass sie in axialer Richtung etwas nach hinten, d.h. in eine zum Anschlag 8 entgegengesetzte Richtung, ausweichen kann, da ansonsten die beiden Gewindeabschnitte 12 und 38 ein vollständiges axiales Einführen des Schraubendrehabschnitts 6 in die Schraubendreheraufnahme ohne gleichzeitiges Drehen der Spannhülse 16 bzw. der Klemmhülse 10 behindern würden. Bei axialer Bewegungsfreiheit der Klemmhülse 10 kann in einem ersten Schritt der Schraubendrehabschnitt 6 vollständig eingeführt werden und in einem zweiten Schritt die Klemmhülse 10 in die Tulpe 36 eingeschraubt werden. Die Eingriffsabschnitte der Spannhülse 16 und der Klemmhülse 10 bzw. die Kronen 18 und 14 sind dann vorzugsweise so gestaltet, dass sie einander in axialer Richtung derart überlappen, dass die Klemmhülse 10 axial relativ zur Spannhülse 16 bewegt werden kann, ohne dabei den Eingriff bzw. den Formschluss in Drehrichtung zu verlieren.

Über die Form des Anschlags 8 und dessen Zusammenwirken mit den Flanken der Tulpe 36 erfolgt eine drehfeste Koppelung zwischen Schaft 2 und Tulpe 36, so dass durch Manipulationen am Schaft 2 bzw. an der Klemmhülse 10 neben Kräften ebenso Momente übertragen werden können.

An dem als Kupplung für einen Handgriff dienenden proximalen Mehrkantprofilabschnitt 4 kann ein Handgriff oder eine sonstige Handhabe zum Drehen des Schafts 2 angebracht werden, um dann die mit dem Schraubendreher 1 spielfrei gekoppelte Pedikelschraube in einen Wirbelkörper zu schrauben. Während der Operateur mit der einen Hand den Handgriff dreht, kann er mit der anderen Hand das gesamte Instrument bzw. den Schraubendreher 1 durch Greifen der relativ drehbaren Außenhülse 26 halten und ausrichten, bis die Knochenschraube 32 in der gewünschten Tiefe in den Wirbel 42 eingeschraubt ist. Um das Resultat zu überprüfen, kann zu jedem Zeitpunkt der Handgriff entfernt und die Drehmechanik bestehend aus Spannhülse 16 und Außenhülse 26 demontiert werden, um durch Röntgenaufnahmen den Sitz der Knochenschraube 32 zu überprüfen.

Für eine anschließende Zementaugmenation und/oder Distraktion oder Kompression ist es wichtig, dass die axial feste Verbindung zwischen Schraubendreher 1 und Pedikelschraube 30 beibehalten wird.

Da der Schaft 2 mit einem über dessen gesamte axiale Länge erstreckenden durchgehenden und innenliegenden Kanal 3 versehen ist, kann der Schaft 2 selbst zur Zementaugmentation benutzt werden. Somit kann unmittelbar nach dem Einschrauben der Pedikelschrauben ohne Instrumentenwechsel über den Schaft 2 Zement in die kanülierte Pedikelschraube 30 injiziert werden kann, wozu lediglich ein Zementapplikator an dem proximalen Ende des Schafts 2 bzw. dem Luer-Anschluss 5 angeschlossen werden muss. Alternativ kann zuvor eine Kanüle in den Kanal bzw. Durchgangsbohrung 3 eingeführt werden. Die über die Klemmhülse aufrechterhaltene Verspannung von Schaft 2 und Pedikelschraube 32 hält dem Druck der Zementierung Stand.

Nach dem Setzen von Pedikelschrauben und evtl. Zementieren wird zum Repositionieren oft distrahiert oder komprimiert. Der erfindungsgemäße Schraubendreher 1 ist so konstruiert, dass er eine steife Verbindung zur Knochenschraube herstellt und der Schaft 2 eine Angriffsfläche für alle Arten von Distraktoren / Kompressoren bietet. Um mit den Distraktoren / Kompressoren unmittelbar am Schaft 2 angreifen zu können, lassen sich die Spannhülse 16 und die Außenhülse 26 vom Schaft 2 demontieren, ohne dabei die Verspannung von Schraubendreher 1 und Pedikelschraube 30 aufzugeben. Die Spannhülse 16 und die Klemmhülse 10 sind nicht fest, insbesondere nicht axial, miteinander verbunden, sondern lediglich über die Kronen 14 und 18 in Eingriff. Somit lässt sich die Spannhülse nach dem Lösen der axialen Sicherung über die Arretierungsvorrichtung axial von der Klemmhülse 10 und vom Schaft 2 abziehen, während die Klemmhülse 10 die Vorspannung selbsthemmend aufrechterhält.

Die Konstruktion ist also so ausgelegt, dass der Schraubendreher 1 trotz Demontage beider Hülsen 16 und 26 nach wie vor sicher über die am Schraubendreher 1 verbliebene Klemmhülse 10 verspannt ist und in jede Richtung Kräfte und/oder Drehmomente übertragen werden können, was leichte Derotationen ermöglicht, bei gleichzeitiger freier Sicht auf den OP-Bereich.

Nach dem Entfernen der Spannhülse 16 und der Außenhülse 26 können Distraktoren / Kompressoren, insbesondere Paralleldistraktoren und / oder - kompressoren, am Schaft 2 oder an der mit dem Schaft 2 verspannten Klemmhülse 10 montiert werden, so dass eine spielfreie Distraktion oder Kompression erfolgen kann. Alternativ kann am Schaft 2 eine Kupplung für z.B. CLR-Sperrer vorgesehen sein.

Nach erfolgter Zementierung und/oder Distraktion oder Kompression lässt sich der Schraubendreher 1 von der Pedikelschraube 30 entkoppeln, indem die Spannhülse 16 zusammen mit der Außenhülse 26 wieder axial auf den Schaft 2 aufgeschoben wird und über die Spannhülse 16 und den Eingriff oder Verzahnung der Krone 18 der Spannhülse 16 und der Krone 14 die Klemmhülse 10 wieder aus der Tulpe 16 herausgedreht wird und dabei die Verspannung von Schraubendreher 1 und Pedikelschraube 30 gelöst wird. Anschließend kann der Schraubendreher 1 aus dem Operationsfeld entnommen werden, um anschließend weitere Operationsschritte, wie das Einsetzen eines Verbindungsstabs in die Pedikelschrauben, etc. vornehmen zu können.

Anschließend kann der Schraubendreher wieder demontiert und gereinigt werden. nach erfolgter Zementierung unmittelbar über den Schaft 2, kann der Schaft 2 entsorgt werden und die anderen Bestandteile, d.h. Spannhülse 16, Außenhülse 26, Klemmhülse 10 (falls vom Schaft 2 demontierbar), und Handgriff gereinigt und mit einem neuen Schaft wiederverwendbar sind.

Das erfindungsgemäße Schraubendrehersystem ist nicht auf die zuvor beschriebene Ausführungsform beschränkt.

So kann beispielsweise die Montage von Spannhülse 16 und Außenhülse 26 am Schaft und die damit mögliche Verspannung von Schraubendreher 1 mit der Pedikelschraube 30 auch erst nach dem Einschrauben der Pedikelschraube über Handgriff und Schaft 2 erfolgen. Nach erfolgter Verspannung kann die Spannhülse 16 und Außenhülse 26 zu jedem beliebigen Zeitpunkt demontiert und wieder montiert werden.

Die Krone kann beliebig viele als Zacken ausgebildete Vorsprünge haben, die gegebenenfalls voneinander abweichende Axiallängen aufweisen können, um auf diese Weise ein Koppeln beider komplementärer Eingriffsabschnitte gleichmäßiger und einfacher zu gestalten. Damit wird erreicht, dass nicht alle Zacken gleichzeitig, sondern nacheinander in Eingriff gelangen.

Der Griffabschnitt der Spannhülse hat gemäß dem gezeigten Ausführungsbeispiel eine im Wesentlichen zylindrische Form. Jedoch kann er jede andere Form wie beispielsweise eine Kugelform aufweisen, mit der der Griffabschnitt gut von einem Operateur gegriffen und gedreht werden kann.

Die Kontur der Außenhülse kann ebenfalls jede andere als die gezeigte aufweisen, solange ein Operateur damit das Schraubendrehersystem gut halten und führen kann. So kann ein in Axialrichtung mittlerer Bereich der Außenhülse ballig ausgebildet sein, der besser von einer Hand gegriffen werden kann.

Zwar ist der Schaft mit einem Mehrkantprofil zum Koppeln eines Handgriffs versehen. Dies ist jedoch nicht zwingend und kann gleichermaßen als Keilwellenprofil oder dergleichen ausgeführt sein.

Das Torx-Profil am distalen Schaftende kann abweichend vom gezeigten Schaft als Inbus-, Schlitz- oder Kreuzschlitz-Profil gestaltet sein.

Alternativ kann eine Kanüle durch den Schaft bis zur Knochenschraube eingeschoben werden, mit der der Zement in den Knochen eingespritzt wird. Auf diese Weise kann der Schaft wiederverwendet werden.

Offenbart ist ein Schraubendreher zum Einbringen von monoaxialen und polyaxialen Pedikelschraube, der gleichzeitig die Funktion einer Zementinjektionskanüle erfüllt und zudem als Kraftübertragungsglied dient, an dem Distraktoren und Kompressoren von nahezu jeder Bauart auf einfache Weise gekoppelt werden können. Der Schraubendreher hat eine zweiteilige aus der Spannhülse und Klemmhülse bestehende Zwischenhülse sowie einen einteiligen, durchgängigen Schaft mit einer Durchgangsinnenbohrung. Das ermöglicht es, dass eine verspannte Kopplung des Schraubendrehers mit einer Pedikelschraube während des gesamten Vorgangs des Setzens einer Knochenschraube aufrechterhalten bleiben kann. Zudem können sämtliche Elemente wie die Spannhülse und die Außenhülse jederzeit entkoppelt werden, um die Sicht sowie die Qualität von Röntgenaufnahmen zu verbessern. Des Weiteren kann die Zementaugmentation mit dem als Zementaugmentationshülse fungierenden Schaft im verspannten Zustand vorgenommen werden, und anschließend können Distraktoren und Kompressoren zu Manipulationszwecken, insbesondere bei Parallelmanipulationen, an den verspannten Schaft gekoppelt werden. Auf diese Weise können Kräfte und Momente ohne Instrumentenwechsel und Zeitverlust übertragen werden. Dafür können spezielle Kopplungsgeometrien in Form von Umfangsnuten oder ähnliches am Schaft, vorzugsweise an dessen distalen Bereich, vorgesehen sein.

### Bezugszeichenl iste

Schaft 2
Innenkanal 3
Mehrkantprofil 4
Luer-System 5
Schraubendreherabschnitt (Torx-Profil Eingriffsabschnitt) 6
Anschlag 8
Kegelstumpfförmiger Abschnitt 9
Klemmhülse 10
Kegelstumpfförmiger Abschnitt 11
Außengewinde 12
Abflachung 13
Erster Eingriffsabschnitt 14
Spannhülse 16
Zweiter Eingriffsabschnitt 18
Hülsenabschnitt 19
Griffabschnitt 20
Fügestelle 21
Umfangsnut 22
Druckknopf 24
Vertiefungen 25
Außenhülse 26
Pedikelschraube 30
Knochenschraube 32
Schraubenkopf 34
Tulpe 36
Innengewinde 38
Insert 40
Wirbel 42
Paralleldistraktor / -kompressor 44
Armabschnitte 48a, 48b
Gelenke 49a, 49b
Koppelabschnitte bzw. Greifer 50a, 50b
Erstes Kraftübertragungselement 52a
Zweites Kraftübertragungselement 52b
Erste Führungsschiene 54
Zweite Führungsschiene 56
Kraftaufbringungselement 58

## Patentansprüche

1. Medizinischer Schraubendreher (1) zum Einbringen von mono- oder polyaxialen Pedikelschrauben (30) in einen Wirbelkörper (42), mit:
einem Schaft (2), der im Bereich seines proximalen Endabschnitts einen Kopplungsabschnitt (4) für ein abnehmbares Handhabungsteil zur Drehmomentaufbringung auf den Schaft (2) aufweist und an dessen distalen Endabschnitt ein Schraubendreherabschnitt (6), insbesondere mit einem Torx- oder Inbus-Profil, vorgesehen ist, der in eine entsprechende Aufnahme am Schraubenkopf (34) der Pedikelschraube (30) einführbar ist und ein auf den Schaft (2) aufgebrachtes Drehmoment auf den Schraubenkopf (34) übertragen kann; und
einer um den Schaft (2) relativ drehbaren Hülse (10, 16), deren distaler Endabschnitt einen Kopplungsabschnitt (12), insbesondere Außengewindeabschnitt, zum Koppeln mit einem an einer Tulpe (36) der Pedikelschraube (30) entsprechend ausgebildeten Kopplungsabschnitt (38), insbesondere Innengewindeabschnitt, aufweist,
**dadurch gekennzeichnet, dass**
die Hülse (10, 16) mehrteilig, insbesondere zweiteilig, ausgebildet ist und einen ersten distal angeordneten Hülsenabschnitt (10) mit einem ersten Eingriffsabschnitt (14) und einen zweiten dazu proximal angeordneten Hülsenabschnitt (16) mit einem komplementären zweiten Eingriffsabschnitt (18) aufweist, wobei
der erste Eingriffsabschnitt (14) und der zweite Eingriffsabschnitt (18) derart konfiguriert sind, dass beide miteinander in Eingriff bringbar sind, so dass der zweite Hülsenabschnitt (16) auf den ersten Hülsenabschnitt (10) ein Drehmoment übertragen kann, um den am ersten Hülsenabschnitt (10) vorgesehenen Kopplungsabschnitt (12) mit der Tulpe zu koppeln, und dass der zweite Hülsenabschnitt (16) von dem ersten Hülsenabschnitt (10) in axialer Richtung, insbesondere werkzeuglos, außer Eingriff bringbar ist und von dem Schaft (2) in proximaler Richtung abgezogen werden kann, ohne den ersten Hülsenabschnitt (10) von der Tulpe (36) der Pedikelschraube (30) lösen zu müssen.

2. Medizinischer Schraubendreher (1) nach Anspruch 1, **dadurch gekennzeichnet, dass**
der erste und zweite Eingriffsabschnitt (14, 18) als korrespondierende Kronen ausgebildet sind, die in axialer Richtung in einander greifen und lediglich einen Formschluss in Umfangsrichtung zur Drehmomentübertragung bilden.

3. Medizinischer Schraubendreher (1) nach Anspruch 1 oder 2, **dadurch gekennzeichnet, dass**
der Schaft (2) zwei miteinander montierbare Schaftabschnitte aufweist und der erste Hülsenabschnitt (10) im montierten Zustand zwischen den zwei montierten Schaftabschnitten, insbesondere axial nicht verschiebbar, gefangen ist.

4. Medizinischer Schraubendreher (1) nach einem der vorhergehenden Ansprüche, **dadurch gekennzeichnet, dass**
der erste Hülsenabschnitt (10) am Schaft (2) zumindest über eine vorbestimmte Länge axial verschiebbar gelagert ist, wobei ein in distaler Richtung vor dem Schraubendreherabschnitt (6) am Schaft (2) vorgesehener Anschlag (8) den axialen Freiheitsgrad des ersten Hülsenabschnitts (10) begrenzt, so dass der erste Hülsenabschnitt (10) in distaler Richtung eine axiale Kraft auf den Schaft (2) übertragen kann, wenn dieser mit der Tulpe (36) der Pedikelschraube (30) gekoppelt wird.

5. Medizinischer Schraubendreher (1) nach einem der vorhergehenden Ansprüchen, **dadurch gekennzeichnet, dass**
eine relativ zur Hülse drehbare Außenhülse (26) zum Greifen des Schraubendrehers (1) auf den ersten und/oder zweiten Hülsenabschnitt (10, 16) lösbar aufbringbar, insbesondere aufschiebbar ist.

6. Medizinischer Schraubendreher (1) nach Anspruch 5, **dadurch gekennzeichnet, dass**
die Außenkonturen des ersten Hülsenabschnitts (10) und des zweiten Hülsenabschnitts (16) derart ausgestaltet sind, dass die relativ dazu drehbare Außenhülse (26) zwischen beiden Hülsenabschnitten (10, 16) axial gefangen ist.

7. Medizinischer Schraubendreher (1) nach einem der vorhergehenden Ansprüchen, **dadurch gekennzeichnet, dass**
der Schaft (2) in Axialrichtung einen durchgehenden, innen liegenden Kanal (3) aufweist; und
am proximalen Endabschnitt des Schafts (2) ein Verbindungssystem (5), vorzugsweise ein Luer-System, zum Verbinden eines Zementapplikators mit dem Kanal (3) vorgesehen ist.

8. Medizinischer Schraubendreher (1) nach einem der vorgehenden Ansprüche, **dadurch gekennzeichnet, dass**
der zweite Hülsenabschnitt (16) am Schaft (2) zur axialen Sicherung lösbar arretierbar ist, insbesondere ein am zweiten Hülsenabschnitt (16) vorgesehenes in radialer Richtung vorgespanntes Riegelelement (24) mit einer am Schaft ausgebildeten Umfangsnut (22) selbsttätig in Eingriff kommt und einen axialen Formschluss bildet, der manuell lösbar ist.

9. Medizinischer Schraubendreher (1) nach einem der vorgehenden Ansprüche, **dadurch gekennzeichnet, dass**
eine Drehsperreinrichtung, insbesondere ein Sperrschieber, die zwischen einer Drehposition, in der der zweite Hülsenabschnitt (16) relativ zum Schaft (2) drehbar ist, und einer Sperrposition, in der der zweite Hülsenabschnitt (16) nicht relativ zum Schaft (2) drehbar ist, bringbar ist.

10. Schaft (2) für einen medizinischen Schraubendreher (1) nach einem der vorgehenden Ansprüche, der im Bereich seines proximalen Endabschnitts einen Kopplungsabschnitt (4) für ein abnehmbares Handhabungsteil zur Drehmomentaufbringung auf den Schaft (2) aufweist und an dessen distalen Endabschnitt ein Schraubendreherabschnitt (6), insbesondere mit einem Torx- oder Inbus-Profil, vorgesehen ist, der in eine entsprechende Aufnahme am Schraubenkopf (34) der Pedikelschraube (30) einführbar ist und ein auf den Schaft (2) aufgebrachtes Drehmoment auf den Schraubenkopf (34) übertragen kann, wobei
der Schaft (2) in Axialrichtung einen durchgehenden, innen liegenden Kanal (3) aufweist; und
am proximalen Endabschnitt des Schafts (2) ein Verbindungssystem (5), vorzugsweise ein Luer-System, zum Anschließen eines Zementapplikators an dem Kanal (3) vorgesehen ist,
**dadurch gekennzeichnet, dass**
eine mit einer Tulpe (36) der Pedikelschraube (30) koppelbaren Klemmhülse (10) an dem Schaft (2) derart angeordnet ist, dass die Klemmhülse (10) und der Schaft (2) eine bauliche Einheit bilden.

11. Schaft (2) für einen medizinischen Schraubendreher (1) gemäß Anspruch 10, **dadurch gekennzeichnet, dass**
am distalen Ende der Klemmhülse (10) ein Kopplungsabschnitt (12), insbesondere ein Außengewindeabschnitt, ausgebildet ist, über den der Schaft (2) mit einem an der Tulpe (36) der Pedikelschraube (30) entsprechenden ausgebildeten Kopplungsabschnitt (38), insbesondere ein Innengewindeabschnitt, koppelbar ist.

12. Schaft (2) für einen medizinischen Schraubendreher (1) gemäß einem der Ansprüche 10 oder 11, **dadurch gekennzeichnet, dass**
die Klemmhülse (10) am distalen Ende des Schafts (2) angeordnet ist.

13. Schaft (2) für einen medizinischen Schraubendreher (1) gemäß einem der Ansprüche 10 bis 12, **dadurch gekennzeichnet, dass**
die Klemmhülse (10) am Schaft (2) zumindest über eine vorbestimmte Länge axial verschiebbar gelagert ist, wobei ein in distaler Richtung vor dem Schraubendreherabschnitt (6) am Schaft (2) vorgesehener Anschlag (8) den axialen Freiheitsgrad des ersten Hülsenabschnitts (10) begrenzt.

14. Schaft (2) für einen medizinischen Schraubendreher (1) gemäß einem der Ansprüche 10 bis 13, **dadurch gekennzeichnet, dass**
die Klemmhülse (10) auf dem Schaft (2) aufgeschrumpft ist.

15. Schaft (2) für einen medizinischen Schraubendreher (1) gemäß einem der Ansprüche 10 bis 13, **dadurch gekennzeichnet, dass**
der Schaft (2) zwei miteinander montierbare Schaftabschnitte aufweist und die Klemmhülse (10) im montierten Zustand zwischen den zwei montierten Schaftabschnitten, insbesondere axial nicht verschiebbar, gefangen ist.

## Claims

1. A medical screw driver (1) for introducing mono-axial or poly-axial pedicle screws (30) into a vertebral body (42) comprising:
a shank (2) which includes in the area of its proximal end portion a coupling portion (4) for a detachable handling member to apply a torque to the shank (2) and at the distal end portion of which a screw driver portion (6), especially comprising a Torx or hexagon socket profile, is provided that is adapted to be inserted in a corresponding seat at the screw head (34) of the pedicle screw (30) and which can transmit a torque applied to the shank (2) to the screw head (34); and
a sleeve (10, 16) relatively rotatable about the shank (2) the distal end portion of which includes a coupling portion (12), especially a male thread portion, for coupling with a coupling portion (38), especially a female thread portion, appropriately formed at a head (36) of the pedicle screw (30),
**characterized in that**
the sleeve (10, 16) has a multi-part, especially two-part design and comprises a distally arranged first sleeve portion (10) including a first engaging portion (14) and a second sleeve portion (16) arranged proximally thereto including a complementary second engaging portion (18), wherein
the first engaging portion (14) and the second engaging portion (18) are configured such that both can be made to engage so that the second sleeve portion (16) can transmit a torque to the first sleeve portion (10) so as to couple the coupling portion (12) provided on the first sleeve portion (10) to the head, and that the second sleeve portion (16) can be disengaged from the first sleeve portion (10) in the axial direction, especially without tools, and can be drawn off the shank (2) in the proximal direction without having to release the first sleeve portion (10) from the head (36) of the pedicle screw (30).

2. The medical screw driver (1) according to claim 1, **characterized in that**
the first and second engaging portions (14, 18) are in the form of corresponding crowns which are engaged in the axial direction and merely constitute a form closure in the circumferential direction for torque transmission.

3. The medical screw driver (1) according to claim 1 or 2, **characterized in that**
the shank (2) includes two jointly mountable shank portions and the first sleeve portion (10) is caught, especially while not being axially movable, between the two mounted shank portions in the mounted condition.

4. The medical screw driver (1) according to one of the preceding claims, **characterized in that**
the first sleeve portion (10) is supported to be axially movable on the shank (2) at least over a predetermined length, wherein a stop (8) provided at the shank (2) in the distal direction ahead of the screw driver portion (6) restricts the axial degree of freedom of the first sleeve portion (10) so that the first sleeve portion (10) can transmit an axial force to the shank (2) in the distal direction, when the shank is coupled to the head (36) of the pedicle screw (30).

5. The medical screw driver (1) according to one of the preceding claims, **characterized in that**
an outer sleeve (26) rotatable relative to the sleeve can be detachably applied to, especially pushed onto, the first and/or second sleeve portion (10, 16) for gripping the screw driver (1).

6. The medical screw driver (1) according to claim 5, **characterized in that**
the outer contours of the first sleeve portion (10) and of the second sleeve portion (16) are configured so that the outer sleeve (26) rotatable relative thereto is axially caught between the two sleeve portions (10, 16).

7. The medical screw driver (1) according to one of the preceding claims, **characterized in that**
the shank (2) has a continuous internal passage (3) in the axial direction; and
at the proximal end portion of the shank (2) a connecting system (5), preferably Luer system, is provided for connecting a cement applicator to the passage (3).

8. The medical screw driver (1) according to one of the preceding claims, **characterized in that**
the second sleeve portion (16) can be detachably locked at the shank (2) for axial securing, especially a locking bar (24) biased in the radial direction and provided at the second sleeve portion (16) is automatically engaged in a peripheral groove (22) formed at the shank and forms an axial form closure which is manually detachable.

9. The medical screw driver (1) according to one of the preceding claims, **characterized in that**
a rotary locking means, especially a stop valve, is provided between a rotating position in which the second sleeve portion (16) is rotatable relative to the shank (2) and a locking position in which the second sleeve portion (16) is not rotatable relative to the shank (2).

10. A shank (2) for a medical screw driver (1) according to one of the preceding claims, which in the area of its proximal end portion includes a coupling portion (4) for a detachable handling member for applying a torque to the shank (2) and at the distal end portion of which a screw driver portion (6), especially having a Torx or hexagon socket profile, is provided which is adapted to be inserted in a corresponding seat at the screw head (34) of the pedicle screw (30) and which can transmit a torque applied to the shank (2) to the screw head (34), wherein
the shank (2) includes a continuous internal passage (3) in the axial direction; and at the proximal end portion of the shank (2) a connecting system (5), preferably a Luer system, is provided to connect a cement applicator to the passage (3),
**characterized in that**
a clamping sleeve (10), which can be coupled to a head (36) of the pedicle screw (30), is attached to the shank (2) in such a way that the clamping sleeve (10) and the shank (2) form a structural unit.

11. The shank (2) for a medical screw driver (1) according to claim 10, **characterized in that**
at the distal end of the clamping sleeve (10), a coupling portion (12), especially a male thread, is formed via which the shaft (2) can be coupled to a coupling portion (38), especially a female thread, appropriately formed at the head (36) of the pedicle screw (30).

12. The shank (2) for a medical screw driver (1) according to one of the claims 10 or 11, **characterized in that**
the clamping sleeve (10) is arranged at the distal end of the shank (2).

13. The shank (2) for a medical screw driver (1) according to one of the claims 10 to 12, **characterized in that**
the clamping sleeve (10) can be supported to be axially movable on the shank (2) at least over a predetermined length, with a stop (8) provided in the distal direction ahead of the screw driver portion (6) at the shank (2) limiting the axial degree of freedom of the first sleeve portion (10).

14. The shank (2) for a medical screw driver (1) according to one of the claims 10 to 13, **characterized in that**
the clamping sleeve (10) is shrunk onto the shank (2).

15. The shank (2) for a medical screw driver (1) according to one of the claims 10 to 13, **characterized in that**
the shank (2) includes two shank portions adapted to be jointly mounted and the clamping sleeve (10) is caught in the mounted state between the two mounted shaft portions to be especially not movable in the axial direction.

## Revendications

1. Tournevis médical (1) pour introduire des vis pédiculaires (30) mono- ou polyaxiales dans un corps de vertèbre (42) présentant :
un arbre (2) qui présente dans la zone de sa section d'extrémité proximale une section de couplage (4) pour une pièce de manipulation amovible permettant d'appliquer un couple de torsion à l'arbre (2) et à la section d'extrémité distale duquel il est prévu une section de tournevis (6), en particulier avec un profil Torx ou à six pans creux, qui peut être insérée dans un logement correspondant sur la tête de vis (34) de la vis pédiculaire (30) et peut transférer un couple de torsion appliqué à l'arbre (2) sur la tête de vis (34) ; et
une douille (10, 16) à rotation relative autour de l'arbre (2), dont la section d'extrémité distale présente une section de couplage (12), en particulier une section de filet externe, pour se coupler à une section de couplage (38) conçue de manière correspondante à une tulipe (36) de la vis pédiculaire (30), en particulier une section de filet interne,
**caractérisé en ce que**
la douille (10, 16) est conçue en plusieurs parties, en particulier en deux parties, et présente une première section de douille (10) agencée de manière distale avec une première section de prise (14) et une seconde section de douille (16) agencée de manière proximale à celle-ci avec une seconde section de prise (18) complémentaire, dans lequel
la première section de prise (14) et la seconde section de prise (18) sont configurées de sorte que toutes deux puissent être amenées en prise mutuelle afin que la seconde section de douille (16) puisse transférer un couple de torsion à la première section de douille (10) pour coupler la première section de couplage (12) disposée sur la première section de douille (10) à la tulipe et que la seconde section de douille (16) puisse être amenée par la première section de douille (10) dans la direction axiale, en particulier sans outil, hors prise et puisse être enlevée de l'arbre (2) dans la direction proximale sans devoir libérer la première section de douille (10) de la tulipe (36) de la vis pédiculaire (30).

2. Tournevis médical (1) selon la revendication 1, **caractérisé en ce que**
la première et la seconde section de prise (14, 18) se présentent sous la forme de couronnes correspondantes qui s'engrènent l'une dans l'autre dans la direction axiale et forment simplement une fermeture mécanique dans la direction périphérique pour transférer le couple de torsion.

3. Tournevis médical (1) selon la revendication 1 ou 2, **caractérisé en ce que**
l'arbre (2) présente deux sections d'arbre montables l'une avec l'autre et la première section de douille (10) est retenue à l'état monté entre les deux sections d'arbre montées, en particulier de manière à ne pas pouvoir être déplacée axialement.

4. Tournevis médical (1) selon l'une quelconque des revendications précédentes, **caractérisé en ce que**
la première section de douille (10) est montée sur l'arbre (2) de manière à pouvoir être déplacée axialement au moins sur une longueur prédéterminée, dans lequel une butée (8) disposée dans la direction distale devant la section de tournevis (6) sur l'arbre (2) délimite le degré de liberté axial de la première section de douille (10) de sorte que la première section de douille (10) puisse transférer à l'arbre (2) une force axiale dans la direction distale lorsque celle-ci est couplé à la tulipe (36) de la vis pédiculaire (30).

5. Tournevis médical (1) selon l'une quelconque des revendications précédentes, **caractérisé en ce que**
une douille externe rotative (26) par rapport à la douille peut être appliquée de manière amovible, en particulier soumise à un coulissement, sur la première et/ou la seconde section de douille (10, 16) pour saisir le tournevis (1).

6. Tournevis médical (1) selon la revendication 5, **caractérisé en ce que**
les contours externes de la première section de douille (10) et de la seconde section de douille (16) sont conçus de sorte que la douille externe rotative (26) par rapport à ceux-ci soit retenue axialement entre les deux sections de douille (10, 16).

7. Tournevis médical (1) selon l'une quelconque des revendications précédentes, **caractérisé en ce que**
l'arbre (2) présente dans la direction axiale un canal interne ininterrompu (3) ; et
il est prévu sur la section d'extrémité proximale de l'arbre (2) un système de liaison (5), de préférence un système Luer, pour relier un applicateur de ciment au canal (3).

8. Tournevis médical (1) selon l'une quelconque des revendications précédentes, **caractérisé en ce que**
la seconde section de douille (16) peut être bloquée de manière amovible sur l'arbre (2) à des fins de fixation axiale, en particulier un élément de blocage (24) disposé sur la seconde section de douille (16) et précontraint dans la direction radiale vient en prise automatiquement avec une rainure périphérique (22) formée sur l'arbre et forme une fermeture mécanique axiale qui peut être détachée à la main.

9. Tournevis médical (1) selon l'une quelconque des revendications précédentes, **caractérisé en ce que**
un dispositif d'arrêt de rotation, en particulier un registre d'arrêt, qui peut être amené entre une position de rotation, dans laquelle la seconde section de douille (16) peut tourner par rapport à l'arbre (2), et une position de blocage, dans laquelle la seconde section de douille (16) ne peut tourner par rapport à l'arbre (2).

10. Arbre (2) pour un tournevis médical (1) selon l'une quelconque des revendications précédentes, qui présente, dans la zone de sa section d'extrémité proximale, une section de couplage (4) pour une pièce de manipulation amovible afin d'appliquer un couple de torsion à l'arbre (2) et à la section d'extrémité distale duquel il est prévu une section de tournevis (6), en particulier avec un profil Torx ou à six pans creux, qui peut être inséré dans un logement correspondant sur la tête de vis (34) de la vis pédiculaire (30) et peut transférer un couple de torsion appliqué à l'arbre (2) à la tête de vis (34), dans lequel
l'arbre (2) présente dans la direction axiale un canal interne ininterrompu (3) ; et
il est prévu dans la section d'extrémité proximale de l'arbre (2) un système de liaison (5), de préférence un système Luer, pour raccorder un applicateur de ciment au canal (3),
**caractérisé en ce que**
une douille de blocage (10) qui peut être couplée à une tulipe (36) de la vis pédiculaire (30) est agencée sur l'arbre (2) de sorte que la douille de blocage (10) et l'arbre (2) forment une unité structurelle.

11. Arbre (2) pour un tournevis médical (1) selon la revendication 10, **caractérisé en ce que**
à l'extrémité distale de la douille de blocage (10) est formée une section de couplage (12), en particulier une section de filet externe, par laquelle l'arbre (2) peut être couplé à une section de couplage (38) conçue de manière correspondante à la tulipe (36) de la vis pédiculaire (30), en particulier une section de filet interne.

12. Arbre (2) pour un tournevis médical (1) selon l'une quelconque des revendications 10 ou 11, **caractérisé en ce que**
la douille de blocage (10) est agencée à l'extrémité distale de l'arbre (2).

13. Arbre (2) pour un tournevis médical (1) selon l'une quelconque des revendications 10 à 12, **caractérisé en ce que**
la douille de blocage (10) est montée sur l'arbre (2) de manière à pouvoir être déplacée axialement au moins sur une longueur prédéterminée, dans lequel une butée (8) aménagée sur l'arbre (2) dans la direction distale avant la section de tournevis (6) délimite le degré de liberté axial de la première section de douille (10).

14. Arbre (2) pour un tournevis médical (1) selon l'une quelconque des revendications 10 à 13, **caractérisé en ce que**
la douille de blocage (10) est frettée sur l'arbre (2).

15. Arbre (2) pour un tournevis médical (1) selon l'une quelconque des revendications 10 à 13, **caractérisé en ce que**
l'arbre (2) présente deux sections d'arbre qui peuvent être montées l'une avec l'autre et la douille de blocage (10) est retenue à l'état monté entre les deux sections d'arbre monté, en particulier de manière à ne pas pouvoir être déplacée axialement.
